# EUROPEAN PATENT APPLICATION

(11) **EP 3 391 857 A1**
(43) Date of publication of application: **24.10.2018**
(21) Application number: 18166809.6
(22) Date of filing: 11.04.2018
(51) Int. Cl.: A61F 2/24, A61M 25/00

(54) **CATHETER DEVICE COMPRISING ROTATABLE DISTAL TIP**

(30) Priority: 21.04.2017 DE 102017108521
(71) Applicant: BIOTRONIK AG, 8180 Bülach (CH)
(72) Inventor: Rothstock, Stephan, 13353 Berlin (DE)
(74) Representative: Keck, Hans-Georg

(57) **Abstract**

The invention relates to a catheter device (1) for transporting an expandable prosthesis (2) to a target site in a body lumen of a patient, comprising a rotatable catheter tip.

## Description

The invention relates to a catheter device comprising a distal tip which is suitable to align a prosthesis located thereon according to the anatomical circumstances.

Such catheter devices are used to transport an expandable prosthesis to the target site in a body lumen of a human patient, or potentially an animal patient. Such a prosthesis may be a stent, for example, or a prosthesis comprising such a stent as a carrier, for example a heart valve prosthesis comprising a heart valve made of biological tissue.

A catheter device of the type mentioned above can comprise an inner shaft extending in an axial direction and an outer shaft extending in the axial direction and surrounding the inner shaft at least in sections for the minimally invasive implantation of the prosthesis, wherein the outer shaft comprises a distal end section, which forms a sheath for receiving or for transporting the prosthesis, wherein the distal end section or the sheath of the outer shaft can furthermore be configured to be axially displaced with respect to the inner shaft so as to expose the prosthesis. The prosthesis may thus simultaneously be released and fixed or implanted as intended at the target site.

Such catheter devices comprising a continuous outer shaft and a core, displaceable with respect thereto, in the form of an inner shaft generally require significant force expenditure on the part of the surgeon to be rotated, and verification of the anatomically correct angular position can, if at all, only take place radiologically by way of X-ray images. A determination of the position of the catheter (angular position) relative to anatomical structures (landmarks), such as the heart valves or the sinuses (bulbs), is possible only to a very limited degree by way of X-ray images due to the absent contrast. Such positionings are theoretically only possible by way of CT images (or MRI images) and 3D models of the human annulus and cardiac tract.

US 2007/0260263 A1 describes a catheter system comprising a rotatable tip. This system, however is aligned such that the rotation of the tip cannot be controlled by the surgeon, and it is not possible to influence the orientation of the tip from the outside.

Proceeding therefrom, it is the object of the present invention to improve a catheter device of the type mentioned above.

This object is achieved by a catheter device having the features of claim 1. Advantageous embodiments are provided in the dependent claims and will be described hereafter.

According to the invention, a catheter device for transporting a preferably expandable prosthesis is provided, comprising:
- an inner shaft extending along a longitudinal axis;
- a catheter tip for carrying the prosthesis;
- wherein, according to the invention, the catheter tip is connected rotatably in at least one direction of rotation to a distal end of the inner shaft so that the catheter tip can be rotated about the longitudinal axis in the at least one direction of rotation with respect to the inner shaft, a plurality of extendable elements being provided at the catheter tip for aligning the catheter tip in the direction of rotation, which when extended project from the catheter tip, for example along the longitudinal axis (which is to say radially outwardly).
The extendable elements should have a tripartite symmetrical composition and be shaped such that a propeller effect is avoided.

In an advantageous embodiment, the catheter tip comprises a moment sensor (a torque sensor), which allows the surgeon to obtain quantitative information about the degree of rotation and the rotatory forces acting on the catheter tip. In keeping with this information, the surgeon is able to rotate the catheter, in addition to the self-alignment of the catheter tip, in such a way that the rotatory moment at the catheter tip is zero, approximately zero, or very low. Rotatory forces can arise as a result of the flow resistance of the tripartite symmetrical flow field behind the heart valve or as a result of closing forces of the heart valves on extendable elements at the catheter tip. For this purpose, it is furthermore advantageous that the catheter tip comprises a fixing means, which is configured to suppress a rotation of the catheter tip about the longitudinal axis, so that the catheter tip can be locked in an aligned state. Such a fixing means can be present in the form of a coupling and may be suitable for causing a force transmission to the rotatable catheter tip. In one embodiment, the fixing means can suppress the free rotatability of the catheter tip by way of frictional engagement. In another embodiment, the free rotatability of the catheter tip can be suppressed by way of form fit, in which preferably two elements interlock, for example when the proximal part is not freely rotatable and prevents the distal rotatable part from being freely rotatable as a result of the interlock. In a further preferred embodiment, the free rotatability may be limited in that a spring element works against the free rotation, whereby the rotation can be kept within preferred boundaries.

One advantage of a system in which the catheter tip can be locked in a particular rotational state about the longitudinal axis due to the presence of fixing means is that the user of the device (the surgeon) has greater control over the device and therefore over the implantation procedure. The fixing means indeed ensures that the prosthesis does not rotate during the release procedure of the prosthesis into the body lumen after a specific orientation has previously been set by the surgeon. A further advantage is that the rotational orientation of the tip can be locked into an aligned state relative to feature of the body lumen that is not located at the implantation site itself. One example in which this possibility is particularly advantageous is for the implantation of an artificial (aortic) heart valve. The catheter tip can be rotationally oriented for example by three extendable elements that fit into the sinus bulbs and the rotation subsequently suppressed by the fixing means in this rotational state. The catheter can then be further advanced into the heart towards the implantation position, at which the prosthesis is to be released. Since the orientation is fixed, the surgeon can be sure that the prosthesis is implanted in the rotational orientation previously determined, even when the extendable elements no longer interact with the sinuses.

Due to the combination of the moment sensor and the information provided thereby on the one hand, and the option to prevent or restrict the free rotation as needed on the other, the surgeon can achieve an optimized rotatory alignment of the catheter tip, and thus of an implant to be implanted, in a very elegant and simple manner.

In this way, the invention enables rotatory and anatomically correct positioning of the distal catheter tip, or of the prosthesis fixed thereon, relative to anatomical structures, and more particularly without additional imaging in this regard.

Within the meaning of the present invention, distal shall be understood to mean that a corresponding distal component, a distal section or a distal end in the longitudinal direction of the inner shaft, along which the longitudinal axis of the inner shaft extends, is located further away from a hand grip or a user (physician) of the catheter device than a proximal component, a proximal section or a proximal end.

Furthermore, the catheter device, as is known, can comprise an outer shaft extending in the direction of the longitudinal axis, which surrounds the inner shaft at least in sections, wherein the outer shaft comprises a distal end section, which is configured to receive the prosthesis, wherein the distal end section of the outer shaft is furthermore configured to be axially displaced with respect to the inner shaft (which is to say along the longitudinal axis), so that the prosthesis is exposed. The distal section of the outer shaft is preferably designed in the form of a capsule.

According to one embodiment of the invention, it is particularly preferably provided that the catheter device comprises three, and more particularly exactly three extendable elements. Furthermore, however, it is also provided that the catheter device comprises a number of extendable elements deviating from three, if this appears to be anatomically expedient. For example, the catheter device can also comprise two extendable elements, for example for a mitral valve prosthesis. The catheter device disclosed herein can thus take anatomically different requirements into account via the number of extendable elements, and is therefore advantageously flexible to use.

According to one embodiment, it is provided that the three elements, based on the respective extended state, project from the catheter tip in a circumferential direction of the catheter tip at a distance of 120° (±2°) from one another. The circumferential direction extends around the longitudinal axis of the inner shaft or of the catheter tip in a plane perpendicular to the longitudinal axis. In a further embodiment, which is suitable for a tricuspid valve prosthesis, for example, an arrangement having two angles > 120° and one angle < 120° may be advantageous, wherein the two angles > 120° are approximately or exactly the same in size. Furthermore, the catheter tip can be configured such that only rotations in the range of 60° (±2°) in a circumferential direction are allowed so as to enable a precise adaptation to the anatomy of the patient. Such an embodiment is advantageous, in particular, for the implantation of an aortic valve with the aid of the aortic bulb.

Moreover, according to one embodiment of the invention it is provided that the extendable elements are configured, when extended, to control the positioning of the prosthesis to be implanted, together with the catheter tip. The extendable elements can be implemented in different forms. The extendable elements are preferably present in the form of balloon elements, fins, keels, flappers, wing elements or other shapes suitable for acting as flow resistance elements, while preferably predefining an alignment of the rotatable catheter tip. In one embodiment, the rotatable catheter tip carries extendable elements, which are configured so as to be rotated by the closing forces of a heart valve, and in particular an aortic valve, about the longitudinal axis along the least resistance into a target angle position. The extendable elements can furthermore be configured so as to assume such a radial extent that they interact with the vessel walls, without excessively impairing the rotation. In such an embodiment, the circumstances are advantageously such that it is not only possible to align the catheter tip by way of rotation based on the anatomical conditions or the prevailing flow profile, but that additionally, as a result of light, occasionally interrupted contact with the vessel walls, it can be ensured that the catheter tip, and thus also the prosthesis to be implanted, can be arranged centrally or centrosymmetrically in the center of the vessel, despite laterally directed tension of the catheter, caused by the bend of the aortic arch against which the rigidity of the catheter works. In this way, it is possible to carry out a central implantation that is adapted to the anatomic circumstances.

According to a further embodiment of the invention, it is provided that the elements are configured, during extension into the extended state, to be rotated together with the catheter tip along the least resistance about the longitudinal axis by engagement (anatomical fit) in the three aortic sinuses. This embodiment, in particular, has the advantage that the implant can be guided centrally inside the vessel, even if a central position is less likely to be predefined by the bend of the aortic arch or another bend of a vessel due to the rigidity of the catheter.

According to a further embodiment of the invention, it is provided that the elements are configured to engage in the aortic sinuses in a form-fit manner during the extension, so that the catheter tip is automatically rotated about the longitudinal axis into an anatomic target angle position.

Furthermore, according to one embodiment of the invention it is provided that the elements are configured, when extended, to form flow resistance elements that interact with the flow field of a heart valve, and in particular an aortic valve, so that a rotatory force is exerted at the elements, which rotates the catheter tip into an anatomic target angle position.

In particular with respect to the interaction with a native heart valve, and in particular an aortic valve, or the engagement in the bulbs (aortic sinuses), it is provided according to one embodiment that each element is designed as a balloon element, which is configured to be transferred into the extended position by being inflated. Balloon elements are particularly preferred, since they can be reversibly extended, for example until locking with the fixing means has taken place. The balloons can then again be deflated, which reduces the chances of the balloons interfering with the release of the prosthesis.

As an alternative, in particular with respect to the interaction with the flow field of a heart valve, and in particular an aortic valve, it is provided according to one embodiment of the invention that each element is designed to be transferred into the extended position by way of deformation. Each element can be designed to be self-unfolding or self-extending, which is to say it is able to automatically move or deform into the extended position. For this purpose, the elements can be made of a superelastic material, for example. The self-unfolding or self-extending elements are preferably made of a nickel titanium alloy, and in particular Nitinol.

According to one embodiment of the invention, it is furthermore provided that the catheter device comprises a grip segment, preferably in the form of a hand grip, which is preferably connected to a proximal end of the inner shaft, so that the inner shaft can be guided or moved in the patient's body by way of the hand segment.

The grip segment, and thus the catheter device, furthermore preferably comprises a means for retracting a pulling element (or multiple pulling elements), so that the at least one pulling element displaces (which is to say pulls away from the prosthesis in the proximal direction, for example) the distal end section of the outer shaft, preferably in the form of a capsule, in the longitudinal direction with respect to the inner shaft, whereby the prosthesis is exposed or released. The means can be configured, for example, to roll up the at least one pulling element, or the multiple pulling elements, and thereby retract the same.

Furthermore, the catheter device can be configured so that a partially released prosthesis is moved back beneath the outer shaft (resheathing).

According to one embodiment of the invention, it is furthermore provided that the catheter device comprises the prosthesis, and in particular a heart valve prosthesis, which is configured and provided to replace a defective heart valve, and in particular an aortic valve, but also a mitral valve or a triscupid valve of a patient.

Moreover, according to one embodiment the prosthesis can include a stent or be designed as a stent. The stent is preferably expandable by way of a separate means (for example, by way of an unfoldable balloon) or configured to self-expand (for example, caused by a material property of the stent, such as an elastic restoring force or superelasticity). In the case of a heart valve prosthesis, this stent may be a carrier for a heart valve, which is preferably made of a biological material or tissue.

Further features and advantages of the invention will be described in the description of exemplary embodiments of the invention, based on the figures. In the drawings:
- FIG. 1: shows a schematic view of a catheter device in approximately the entire length (FIG. 1A), with an enlargement of the distal region (FIB. 1B), and with an enlargement of the catheter tip (FIG. 1C);
- FIG. 2: shows a schematic view of a rotatably mounted catheter tip;
- FIG. 3: shows a schematic view of a rotatably mounted catheter tip including inflatable elements for the rotatory alignment of the catheter tip, when not inflated;
- FIG. 4: shows the balloon-like elements according to FIG. 3 when inflated;
- FIG. 5: shows a rotatory alignment of the catheter tip as a result of the interaction with the valvular cusps of the heart valve (FIG. 5B) and an alignment of the catheter tip with the aortic bulbs (aortic sinuses) (FIG. 5C):
- FIG. 6: shows a schematic view and a cross-sectional view of three extendable elements for interaction with the flow or velocity field according to FIG. 8;
- FIG. 7: shows a modification of the elements or flow resistance elements shown in FIG. 6;
- FIG. 8: shows an assumed flow or velocity field of the blood flow on an aortic valve with the valve almost closed;
- FIG. 9: schematically shows two couplings, which can act as fixing means, once in the embodiment using frictional engagement (FIG. 9A) and once in the embodiment using form fit (FIG. 9B); and
- FIG. 10: schematically shows a swivel joint for the rotational mounting of the rotatable tip.

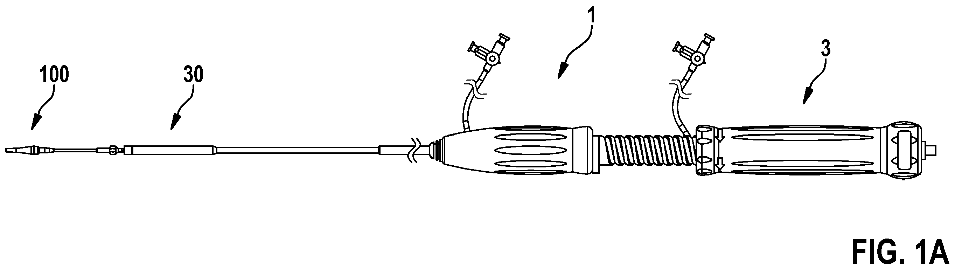

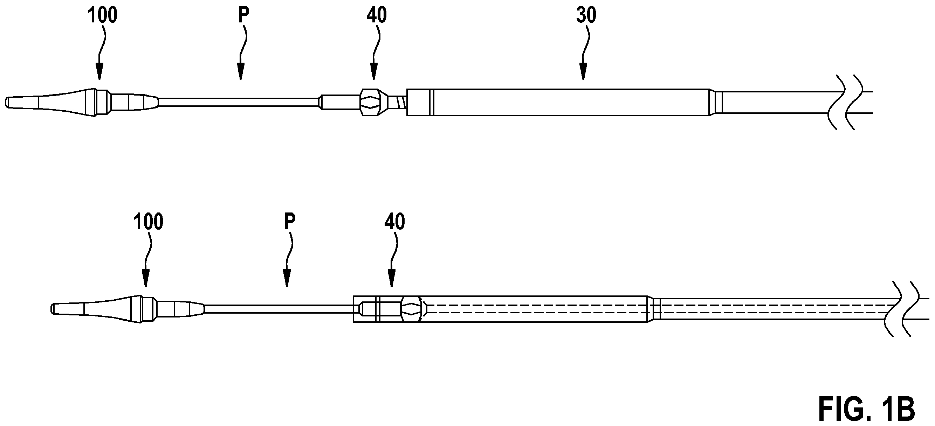

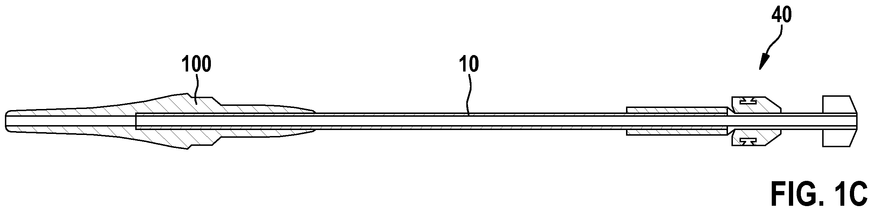

FIGS. 1A-C show the composition of a catheter device for a prosthesis according to the prior art. FIG. 1A shows the catheter device in approximately the entire length. The catheter device comprises a grip segment 3 at the proximal end of the catheter device, a capsule 30, and a catheter tip 100 at the distal end. FIG. 1B shows two views of the schematic illustration of the distal region of the catheter device. The distal region comprises a capsule 30, a connector region 40, a region P in which the prosthesis to be inserted can be guided, and a catheter tip 100. The region P is delimited by the proximal end of the catheter tip 100 and the distal region of the connector region 40. FIG. 1C shows a further enlargement of the distal region, including the distal tip 100, the inner shaft 10 extending in the region P, and the connector region 40.

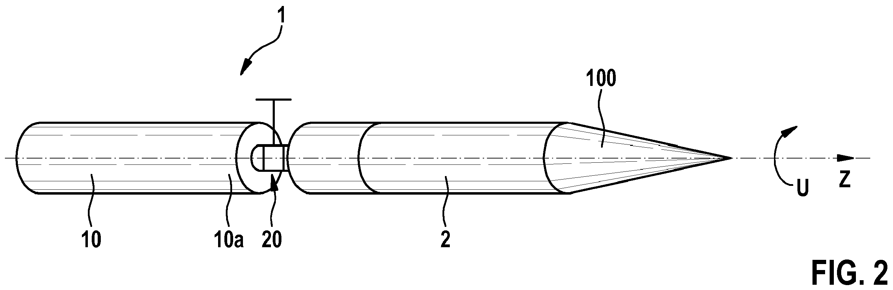

FIG. 2 shows a sectional illustration of a catheter device 1 for transporting an expandable prosthesis 2 to a target site in a body lumen of a patient, wherein the prosthesis 2 is preferably a heart valve prosthesis, which can include a stent, for example, as a carrier for a (for example, biological) heart valve, and in particular an aortic valve. The stent or carrier can be designed as a self-expanding stent or carrier, for example. The catheter device 1 furthermore comprises a flexible inner shaft 10 extending along a longitudinal axis z (also referred to as axial direction z), and an outer shaft (not shown in FIG. 2) extending along the longitudinal axis z, which surrounds the inner shaft 10 at least in sections, wherein the outer shaft can comprise a distal end section configured to receive the prosthesis 2, which is disposed on a catheter tip 100 provided at the distal end of the inner shaft 10, and to hold the same in a compressed state, wherein the distal end section of the outer shaft can furthermore be configured to be displaced with respect to the inner shaft 10 in the axial direction z, so that the prosthesis 2 is exposed and thereby unfolds autonomously into an expanded state at the target site.

So as to also create the option to be able to rotate the prosthesis automatically about the longitudinal axis z of the inner shaft 10 into an anatomically desired target angle position, so that, for example, a prosthesis 2 that is not rotation-symmetrical with respect to the longitudinal axis z can be correctly positioned at the target site, it is provided according to the invention that the catheter tip 100 is coupled rotatably in a direction of rotation to the distal end of the inner shaft 10 so that the catheter tip 100 can be rotated about the longitudinal axis z in the direction of rotation with respect to the inner shaft 10, wherein a plurality of extendable elements 20 is provided at the catheter tip 100 for aligning the catheter tip 100 in the direction of rotation, which, when extended, each project preferably perpendicularly from the catheter tip 100 along the longitudinal axis z.

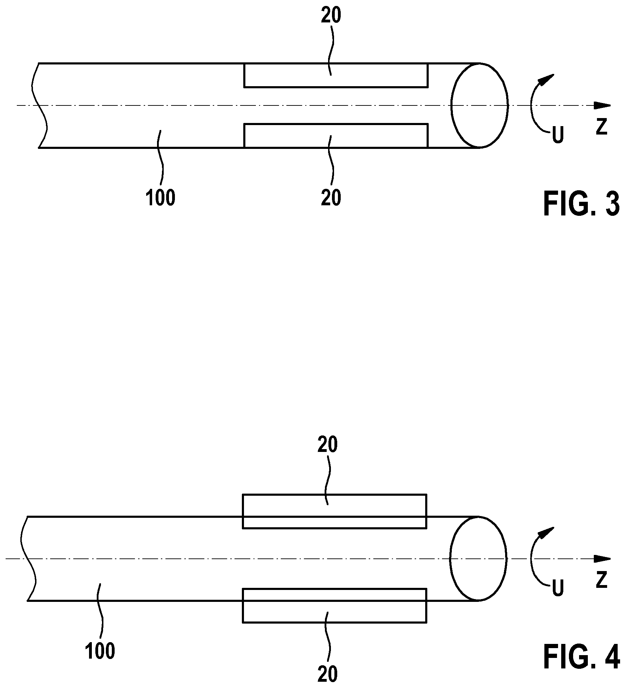

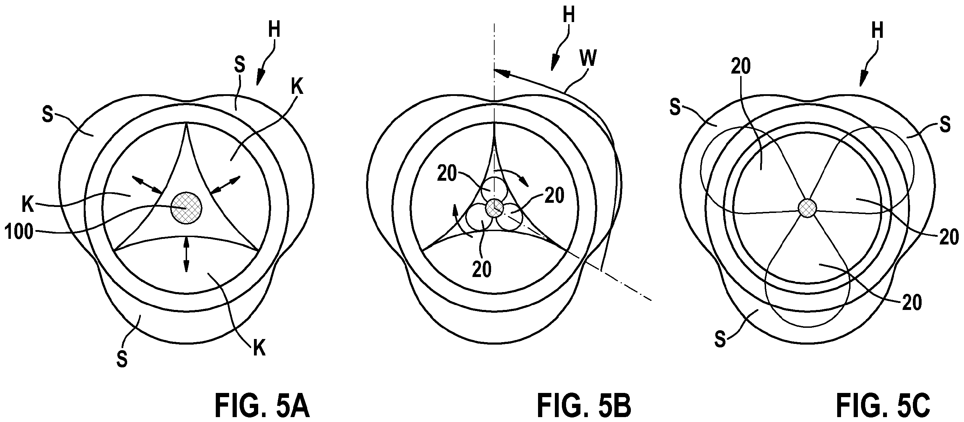

As shown in FIGS. 3 to 7, the elements 20 are disposed at an angular distance of preferably 120° with respect to one another in the direction of the circumference U of the catheter tip 100, which is to say two radius vectors, which originate from the (central) longitudinal axis z of the catheter tip 100 and extend toward two adjoining extendable elements 20, in each case form an angle of 120° with one another, as is indicated in FIGS. 5B, 6 and 7. Such a distribution of the elements 20 along the circumference U of the catheter tip is also referred to as tripartite symmetrical herein. Furthermore, the positions of the extendable elements 20 may deviate from a tripartite symmetrical arrangement, if this is anatomically expedient. An arrangement having two angles > 120° and one angle < 120° may be advantageous for a tricuspid valve prosthesis. However, it may also be desirable for a different number of extendable elements 20 to be provided at the catheter tip 100 in the circumferential direction. For the use of a mitral valve prosthesis, for example, an embodiment comprising only two extendable elements 20 may be desirable.

FIGS. 5 show the approximation and adaptation of the rotatable catheter tip 100 with respect to a heart valve H in a front view. FIG. 5A shows three valvular cusps K, which can open and close according to the directions of the arrows. The bulbs S are indicated laterally behind the valvular cusps K. The catheter tip 100 is still shown without extendable elements 20 in FIG. 5A. According to one embodiment of the invention (see FIG. 5B), it is now provided that the distal catheter tip 100 mechanically aligns with the anatomical structures at the site of the implantation, or the vicinity thereof, in the direction of rotation. For example, the distal catheter tip 100 is rotated into the desired position by the extendable elements 20 in the form of inflatable balloon elements based on the closing forces of the heart valve H or valvular cusp K (see FIG. 5B).

As an alternative, the balloon elements can be rotated about the longitudinal axis z together with the catheter tip 100 based on the geometric shape of the bulbs S, and thus be brought into the target position (FIG. 5C). It may be provided that the balloon elements, when extended, engage in the bulbs (for example by way of form fit). It is furthermore also apparent from this illustration that the balloon elements help to dispose the catheter tip 100 centrally in the middle of the vessel, despite laterally directed tension of the catheter caused by the bend of the aortic arch.

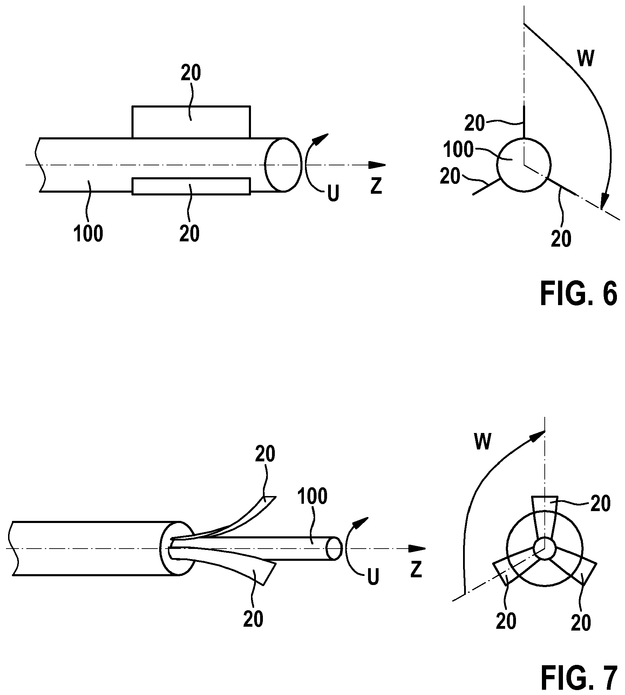

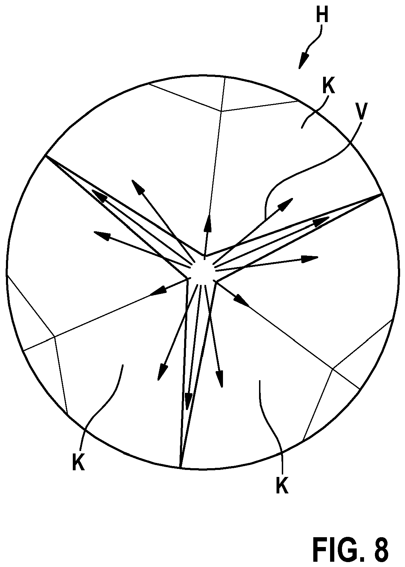

According to a further exemplary embodiment (see FIGS. 5, 6 and 8), a rotatory alignment of the catheter tip 100 based on the flow resistance of the elements 20 is provided, which preferably form extendable flow resistance elements (FIGS. 5 and 6). When the valve H is being closed, preferred regions in terms of fluid mechanics having high and low flow velocities V are created, which in the case of an aortic valve have an approximately tripartite symmetrical distribution. This is illustrated by arrows in FIG. 8. The lengths of the arrows represent an estimation and are to be validated by fluid mechanics analysis. Furthermore, long arrows could indicate a high flow velocity, and short arrows could indicate a low flow velocity. When an element 20 having a favorable shape in terms of fluid mechanics is now located at the catheter tip 100 (for example, tripartite symmetrically as described above, which is to say W = 120°), this element can align along the least flow resistance. It is expected that the flow field V changes dynamically during the closing of the heart valve H, and that maximum flow velocities and gradients prevail just prior to closing, which rotate the distal catheter tip 100 along the least resistance.

According to FIGS. 5 and 6, the elements 20 or flow resistance elements can be designed as wings or fins, which project from the catheter tip perpendicularly to the flow direction and, as described above, are again disposed in a tripartite symmetrical manner. As was likewise described above, however, other arrangements may also be implemented.

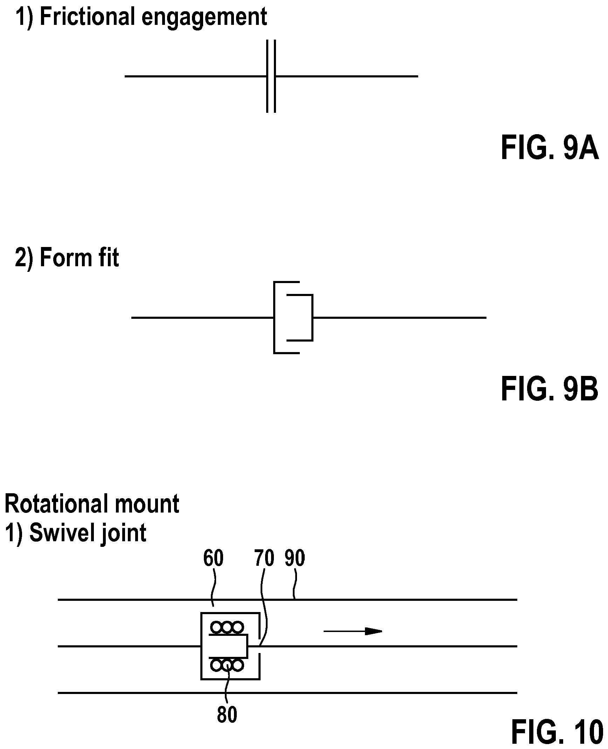

In the above-described embodiments, the catheter tip 100 is mounted so as to rotate freely about the longitudinal axis, wherein a fixing means 50 is preferably provided, by way of which a rotation of the catheter tip about the longitudinal axis can be suppressed, so that the catheter tip is lockable in an aligned position with respect to the rotation about the longitudinal axis z. Two embodiments are shown schematically in FIGS. 9A and 9B. In a first embodiment according to FIG. 9A, the fixing means 50 can be implemented by way of frictional engagement, wherein the free rotation is suppressed by the friction of two elements that are pressed against one another, such as plates. The fixing means 50, however, can also comprise an embodiment not based on friction. For this purpose, the fixing means can comprise a lockable gear mechanism, for example, as is shown schematically in FIG. 9B, in which the distal and proximal parts of the fixing means engage in one another, for example, thereby interlocking and thus suppressing the free rotation of the catheter tip 100.

FIG. 10 shows a swivel joint 60 comprising a coupling region 70 and a pivot bearing 80 according to one embodiment. In the coupling region, a force transmission for preventing the free rotation of the rotatable catheter tip 100 is achieved, for example, by pulling on the proximal part of the swivel joint in the direction of the arrow. The pivot bearing 80 can be configured in the form of a ball bearing having a seal. In this illustration, the swivel joint 60 is shown inside the outer shaft 90.

In summary, the solution according to the invention has the advantage that, using slightly modified standard catheters, for example, a rotatory target position of a cardiac catheter relative to anatomical cardiac structures can be achieved without additional radiological measures. In this way, heart valve prostheses and stents (carrier systems for heart valves) can have a tripartite symmetrical design and be anatomically adapted. Moreover, an anatomically correct angular position of TAVI stents can also improve the sealing properties of prostheses.

### List of Reference Signs:

- 1: catheter device
- 2: prosthesis
- 3: grip segment
- 10: inner shaft
- 20: extendable elements
- 30: capsule
- 40: connector region
- 50: fixing means
- 60: swivel joint
- 70: coupling region
- 80: pivot bearing
- 90: outer shaft
- 100: catheter tip
- U: circumferential direction
- W: angle
- H: heart valve
- K: valvular cusp
- V: flow field
- S: bulbs

## Claims

1. A catheter device (1) for transporting a prosthesis (2) to a target site in a body lumen of a patient, comprising:
- an inner shaft (10) extending along a longitudinal axis (z); and
- a catheter tip (100) for carrying the prosthesis (2),
**characterized in that**
the catheter tip (100) is rotatably connected to a distal end of the inner shaft (10) such that the catheter tip (100) can be rotated about the longitudinal axis (z) in at least one direction of rotation with respect to the inner shaft (10), a plurality of extendable elements (20) being provided at the catheter tip (100) for aligning the catheter tip (100) in the direction of rotation which, when extended, project from the catheter tip (100), and the catheter device (1) comprises a fixing means, which is configured to suppress a rotation of the catheter tip (100) about the longitudinal axis (z), so that the catheter tip (100) can be locked in an aligned state.

2. The catheter device according to claim 1, **characterized in that** three extendable elements (20) are provided.

3. The catheter device according to claim 2, **characterized in that** the three elements (20), based on the respective extended state, are disposed in a circumferential direction (U) of the catheter tip (100) at an angular distance of 120° (±2°) from one another and project from the catheter tip (100).

4. The catheter device according to any one of the preceding claims, **characterized in that** the catheter tip comprises a moment sensor, which is able to quantify the rotatory forces prevailing at the catheter tip.

5. The catheter device according to any one of the preceding claims, **characterized in that** the elements (20), when extended, are configured so as to be rotated together with the catheter tip (100) by the closing forces of an aortic valve (H) about the longitudinal axis (z) along the least resistance into a target angle position.

6. The catheter device according to any one of claims 1 to 4, **characterized in that** the elements (20) are configured, during extension into the extended state, to be rotated together with the catheter tip (100) along the least resistance about the longitudinal axis (z) by engagement of the elements (20) in the three aortic sinuses (S).

7. The catheter device according to claim 6, **characterized in that** the elements (20) are configured to engage in the aortic sinuses (S) in a form-fit manner during extension, so that the catheter tip (100) is automatically rotated about the longitudinal axis (z) into a target angle position.

8. The catheter device according to any one of claims 1 to 4, **characterized in that** the elements (20) are configured, when extended, to form flow resistance elements that interact with the flow field (V) of a heart valve (H), so that a rotatory force is exerted at the elements (20), which rotates the catheter tip (100) into an target angle position.

9. The catheter device according to any one of the preceding claims, **characterized in that** each element (20) is designed as a balloon element, which is configured to be transferred into the extended position by being inflated.

10. The catheter device according to any one of claims 1 to 8, **characterized in that** each element (20) is designed to be transferred into the extended position by way of deformation.

11. The catheter device according to claim 10, **characterized in that** each element (20) is designed to be self-extending.

12. The catheter device according to any one of the preceding claims, **characterized in that** the prosthesis (2) is designed as a heart valve prosthesis, which is configured and provided to replace a defective heart valve (H) of a patient.
